# EUROPEAN PATENT APPLICATION

(11) **EP 1 496 315 A1**
(43) Date of publication of application: **12.01.2005**
(21) Application number: 03705406.1
(22) Date of filing: 24.02.2003
(51) Int. Cl.: F24F 11/02

(54) **AIR CONDITIONER, REMOTE CONTROLLER, AND ULTRASONIC WAVE GENERATING UNIT**

(30) Priority: 27.03.2002 JP 2002089149; 06.08.2002 JP 2002228931
(71) Applicant: Daikin Industries, Ltd., Osaka-shi, Osaka 530-8323 (JP)
(72) Inventor: Okamoto, Yoshio, Daikin Ind., Ltd., ShigaPlant, Kusatsu-shi, Shiga 525-0044 (JP); Sakaguchi, T., Daikan Ind., Ltd., Shiga Plant, Kusatsu-shi, Shiga 525-0044 (JP); Yasutomi, Masanao, Daikan Ind., Ltd., Shiga Plant, Kusatsu-shi, Shiga 525-0044 (JP); Ito, Mikio, Daikin Ind., Ltd., Shiga Plant, Kusatsu-shi, Shiga 525-0044 (JP)
(74) Representative: HOFFMANN - EITLE
(86) International application number: PCT/JP2003/002002
(87) International publication number: WO 2003/081139

(57) **Abstract**

The object of the present invention is to provide an air conditioner, a remote controller, and an ultrasonic wave generating unit that are capable of improving the feeling of comfort of room occupants in a room where the air conditioner is used. The air conditioner (1) is an air conditioner that is configured to blow conditioned air into a room and is equipped with an ultrasonic wave generator (6) and a control unit (9). The ultrasonic wave generator (6) is configured to generate ultrasonic waves having frequencies ranging from 20 kHz to 100 kHz. The control unit (9) is configured to control the ultrasonic wave generator (6) such that the ultrasonic waves overlap with the air blowing sound of the air conditioner.

## Description

### Technical Field

The present invention relates to an air conditioner configured to deliver conditioned air into a room, a remote controller configured to send control signals to an air conditioner, and an ultrasonic wave generating unit configured to be mounted to an air conditioner.

### Background Art

An air conditioner serves to improve the comfort of room occupants by delivering conditioned air into the room. For example, the heating/cooling units typically called "air conditioners" can maintain a room temperature that is comfortable for room occupants by delivering cool air or warm air into the room. Meanwhile, air cleaners, another type of air conditioner, serve to keep the air inside a room clean and improve the comfort of room occupants by removing dust, particles, and other contaminants from air drawn into the air cleaner and delivering clean air into the room. Furthermore, in recent years, air conditioners provided with such functions as humidity control, odor removal, and negative ion generation are promoting the comfort of room occupants.

In recent years it has been reported that sounds containing high frequency components exceeding the audible frequency range of the human ear, i.e., exceeding the range from about 20 Hz to 20 kHz, exhibit such an effect (the "hypersonic effect") that they improve the feeling of comfort of a person exposed to them more than sounds that do not include such high frequencies. In particular, such findings have been reported in the following document: Ohashi et al, "Hypersonic Effect," Technical Report of The Institute of Electronics, Information, and Communication Engineers, sp 96 to 112 (1997-02). That document reports that even though individual frequencies in excess of 26 kHz cannot be heard by the human ear, sounds containing frequency components in excess of 26 kHz increase the power spectra of the alpha waves of the brain (measured by electroencephalogram (EEG)) by an amount that is statistically significant in comparison with sounds that do not contain such high-frequency components. Such high-frequency components also have the effect of causing the audible sounds with which they are intermingled to be perceived in a more pleasant manner by the listener. Alpha waves are known as the brain waves whose activity increases when a person closes his or her eyes and relaxes, and it is believed that the hypersonic effect causes a person to experience an increased feeling of comfort.

Sounds that contain high-frequency components that exceed the audible frequency range of the human ear in excess of 20 kHz occur often in natural environments, but such sounds are almost non-existent in indoor spaces that are surrounded by concrete and the like. Thus, in the indoor spaces where the above-mentioned air conditioners are used, such high-frequency containing sounds are almost non-existent and there is little hope of improving comfort by means of the hypersonic effect.

### Disclosure of the Invention

The object of the present invention is to provide an air conditioner, a remote controller, and an ultrasonic wave generating unit that are capable of improving the feeling of comfort of room occupants in a room where the air conditioner is used.

The invention described in claim 1 is an air conditioner that is configured to blow conditioned air into a room and is equipped with an ultrasonic wave generator and a control unit. The ultrasonic wave generator is configured to generate ultrasonic waves having frequencies ranging from 20 kHz to 100 kHz. The control unit is configured to control the ultrasonic wave generator such that the ultrasonic waves overlap with the air blowing sound of the air conditioner.

With this air conditioner, an ultrasonic wave generator is used to generate ultrasonic waves having frequencies ranging from 20 kHz to 100 kHz in such a manner that the ultrasonic waves overlap with the blowing sound of the air conditioner. As a result, the sound that reaches the ears of a room occupant includes the audible blowing sound of the air conditioner and the high-frequency ultrasonic waves having frequencies of 20 kHz and higher. Thus, a room occupant exposed to this sound will experience improved comfort due to the hypersonic effect. Since the attenuation of ultrasonic waves increases as the frequency increases, ultrasonic waves at frequencies exceeding 100 kHz do not easily reach room occupants. However, since the present invention is contrived such that the ultrasonic waves are generated at frequencies ranging from 20 kHz to 100 kHz, the ultrasonic waves can reach the room occupants and can thus impart the hypersonic effect to the room occupants.

In short, an air conditioner in accordance with claim 1 can improve the comfort of room occupants.

The invention described in claim 2 is an air conditioner as described in claim 1, wherein the control unit executes control in such a manner that a characteristic of the ultrasonic waves fluctuates. The ultrasonic wave characteristics mentioned in this invention include the frequency, the sound pressure, and the generation time.

With this air conditioner, the characteristics of the ultrasonic waves generated by the ultrasonic wave generator are made to fluctuate and the comfort of the room occupants is improved due to the effect of the fluctuation.

The invention described in claim 3 is an air conditioner as described in claim 2, wherein the control unit executes control in such a manner that the flow rate or flow speed of the air blown into the room fluctuates and a characteristic of the ultrasonic waves fluctuates at approximately the same cyclical frequency as the fluctuation of the blowing sound of the air conditioner. The control unit can be configured to make the ultrasonic waves fluctuate in an active manner or in a passive manner using the sound wave propagation environment, which varies depending on the flow rate or flow speed of the air blown into the room.

When the cyclical frequencies of the fluctuations of the blowing sound and the ultrasonic waves are different, these sounds are heard as noise and sometimes result in an unpleasant feeling on the part of the room occupants. However, with this air conditioner, a characteristic of the ultrasonic waves is made to fluctuate at approximately the same cyclical frequency as the fluctuation of the blowing sound and, thus, such an unpleasant feeling can be prevented.

The invention described in claim 4 is an air conditioner as described in claim 2 or 3, wherein the characteristic is the frequency of the ultrasonic waves.

With this air conditioner, the frequency of the ultrasonic waves generated by the ultrasonic wave generator is made to fluctuate and the comfort of the room occupants is improved due to the effect of the fluctuation.

The invention described in claim 5 is an air conditioner as described in claim 4, wherein the control unit executes control in such a manner that the frequency of the ultrasonic waves changes at intervals of 1 millisecond, 1 second, or an amount of time between 1 millisecond and 1 second.

Sounds in the natural world include ultrasonic wave components in various frequency bands and these ultrasonic wave components reach the ear simultaneously. A feasible method of creating such a state artificially is to use a plurality of ultrasonic wave generators to generate ultrasonic waves in various frequency bands simultaneously, but this method is expensive because it requires the use of multiple ultrasonic wave generators.

With this air conditioner, however, a state that approximates the natural world can be created inexpensively by making the frequency of the ultrasonic waves change at a high speed such that ultrasonic wave components in various frequency bands can be emitted for short periods of time.

If the interval is shorter than 1 millisecond, it will be difficult to compose a pulse and if the interval is longer than 1 second, the timing at which the frequency of the ultrasonic waves is changed will become slow and it will be difficult to approximate the kind of fluctuation that occurs in the natural world.

The invention described in claim 6 is an air conditioner as described in claim 2 or 3, wherein the characteristic is the sound pressure of the ultrasonic waves.

With this air conditioner, the sound pressure of the ultrasonic waves generated by the ultrasonic wave generator is made to fluctuate and the comfort of the room occupants is improved due to the effect of the fluctuation.

The invention described in claim 7 is an air conditioner as described in claim 6, wherein the ultrasonic wave generator is equipped with an oscillator, an amplifier, and a piezoelectric element. The oscillator is configured to generate a signal having an ultrasonic frequency. The amplifier is configured to amplify the voltage of the signal. The piezoelectric element is configured to convert the amplified signal outputted from the amplifier into ultrasonic waves. The controller varies the amplification factor of the amplifier.

With this air conditioner, the sound pressure fluctuates because the controller varies the amplification factor of the amplifier. As a result, the sound pressure of the ultrasonic waves generated by the ultrasonic wave generator is made to fluctuate and the comfort of the room occupants is improved due to the effect of the fluctuation.

The invention described in claim 8 is an air conditioner as described in claim 4 or 5, wherein the ultrasonic wave generator is equipped with a piezoelectric element. The piezoelectric element has a plurality of sound pressure peaks within the frequency band where the frequency of the ultrasonic waves is made to fluctuate by the control unit.

With this air conditioner, the piezoelectric element provided in the ultrasonic wave generator has a plurality of sound pressure peaks within the frequency band where the frequency of the ultrasonic waves is made to fluctuate by the control unit. Consequently, if the frequency of the ultrasonic waves varies through one round trip across the frequency band, the sound pressure of the ultrasonic waves will also change repeatedly by a number of times equal to the number of peaks. In other words, the sound pressure can be varied several times by merely varying the frequency by a small amount. Thus, the sound pressure can be easily made to fluctuate.

The invention described in claim 9 is an air conditioner as described in any one of claims 2 to 8, wherein the controller has a first control unit and a second control unit. The first control unit controls the air conditioning functions of the air conditioner. The second control unit controls the fluctuation of the ultrasonic waves of the ultrasonic wave generator.

With this air conditioner, the fluctuation of the ultrasonic waves of the ultrasonic wave generator is controlled by a second control unit that is separate from the first control unit that controls the air conditioning functions of the air conditioner. Consequently, the control of the ultrasonic wave fluctuation can be prevented from increasing the load imposed on the first controller used to control the air conditioning functions.

The invention described in claim 10 is an air conditioner as described in any one of claims 1 to 9, wherein the ultrasonic wave generator has a wave transmitter configured to transmit ultrasonic waves toward the inside of the room. The wave transmitter is arranged in a position where it has access to the space inside the room.

Ultrasonic waves do not easily penetrate matter and are readily reflected from the surfaces of matter. Consequently, if the wave transmitter used to transmit ultrasonic waves toward the inside of a room is located deep inside the air conditioner, the components of the air conditioner will act as obstacles causing the ultrasonic waves to attenuate easily. As a result, it will be difficult for the ultrasonic waves to reach the room occupants.

With this air conditioner, however, wave transmitter is arranged in a position where it has access to the space inside the room and the ultrasonic waves are not likely to be hindered by an obstacle before reaching the room occupants. In short, with this air conditioner, the ultrasonic waves easily reach the room occupants.

The invention described in claim 11 is an air conditioner as described in any one of claims 1 to 9, wherein the air conditioner is provided with a casing having a front face part that covers the front face of the air conditioner and the ultrasonic wave generator has a wave transmitter configured to transmit ultrasonic waves toward the inside of the room. The wave transmitter is arranged near the rear side of the front face part.

With this air conditioner, the wave transmitter used to transmit ultrasonic waves toward the inside of the room is arranged near the rear side of the front face part. Thus, the wave transmitter is arranged in one of the more frontward positions inside the air conditioner. As a result, with this air conditioner, there are few objects to obstruct ultrasonic waves transmitted in the forward direction and the ultrasonic waves can more sufficiently reach occupants in the living space in front of the air conditioner.

The invention described in claim 12 is an air conditioner as described in any one of claims 1 to 9, wherein the air conditioner is further provided with a casing and an indoor heat exchanger. The casing has a front face part that covers the front face of the air conditioner. The indoor heat exchanger exchanges heat with the air inside the room. The ultrasonic wave generator has a wave transmitter configured to transmit ultrasonic waves toward the inside of the room. The wave transmitter is arranged between the front face part and the heat exchanger.

With this air conditioner, the wave transmitter used to transmit ultrasonic waves toward the inside of the room is arranged between the front face part and the heat exchanger. Thus, the wave transmitter is positioned farther toward the front than the heat exchanger and is therefore arranged in a position even closer to the front of the air conditioner. As a result, with this air conditioner, there are few objects to obstruct ultrasonic waves transmitted in the forward direction. In short, with this air conditioner, the ultrasonic waves can more sufficiently reach occupants in the living space in front of the air conditioner.

The invention described in claim 13 is an air conditioner as described in any one of claims 10 to 12, wherein the air conditioner is further provided with an outlet vent through which conditioned air is blown out. The wave transmitter is arranged near the outlet vent.

With this air conditioner, however, since the wave transmitter is arranged near the outlet vent, the ultrasonic waves can reach the occupants easily and are not likely to be hindered by an obstacle before reaching the room occupants. Additionally, since the ultrasonic waves are generated in the vicinity of the outlet vent where the blowing sound of the air conditioner occurs, the ultrasonic waves readily overlap with the blowing sound.

The invention described in claim 14 is a remote controller configured to send control signals to an air conditioner as described in any one of claims 1 to 13, the remote controller being provided with a control selecting means configured to allow a user to select how the ultrasonic wave generator will be controlled.

Since the remote controller is provided with a control selecting means configured to allow a user to select how the ultrasonic wave generator will be controlled, a room occupant who wishes to operate the ultrasonic wave generator can easily do so by using the control selecting means.

The invention described in claim 15 is a remote controller configured to send control signals to an air conditioner that is configured as described in any one of claims 2 to 13 and also provided with a memory unit storing a plurality of patterns for fluctuation of the ultrasonic waves and pattern identification data corresponding to each of the patterns, the remote controller being provided with a signal transmitter and a fluctuation pattern selecting means. The fluctuation pattern selecting means allows a user to select the pattern. The signal transmitter transmits pattern recognition data corresponding to the pattern selected using the fluctuation pattern selecting means.

With this remote controller, instead of sending data expressing an entire ultrasonic wave fluctuation pattern, the signal transmitter transmits pattern recognition data corresponding to the selected ultrasonic wave fluctuation pattern. As a result, the volume of data in the transmitted signal is smaller than a case in which the transmitted signal contains data expressing the entire ultrasonic wave fluctuation pattern.

The invention described in claim 16 is an ultrasonic wave generating unit configured to be mounted to an air conditioner that is configured to blow conditioned air into a room, the ultrasonic wave generating unit being equipped with an ultrasonic wave generator and a control unit. The ultrasonic wave generator is configured to generate ultrasonic waves having frequencies ranging from 20 kHz to 100 kHz. The control unit is configured to control the ultrasonic wave generator such that the ultrasonic waves overlap with the air blowing sound of the air conditioner. It is also acceptable for the control unit to be built into the ultrasonic wave generator.

This ultrasonic wave generating unit is a separate unit from the air conditioner and can be mounted to an existing air conditioner. Thus, this ultrasonic wave generating unit makes it possible to add an ultrasonic wave generating function to an existing air conditioner inexpensively.

The air conditioner described in claim 17 is an air conditioner as described in claims 7 or 8, wherein the ultrasonic wave generator is further provided with an audible signal oscillator configured to generate a signal having an audible frequency. The audible signal oscillator can be the same device as the oscillator used to generate ultrasonic frequencies or it can be a separate device.

With this air conditioner, the piezoelectric element used to output the ultrasonic waves can be inspected by generating a signal having an audible frequency and sending the signal to the piezoelectric element. As a result, advanced ultrasonic inspection equipment is not required to inspect the ultrasonic wave generator. This feature enables the equipment used for mass production to be simplified.

### [BRIEF DESCRIPTION OF THE DRAWINGS]

Figure 1 is a diagrammatic view showing the external appearance of an air conditioner.

Figure 2 is a diagrammatic view showing the external appearance of an indoor unit.

Figure 3 is a block diagram showing the constituent features of an ultrasonic wave generator.

Figure 4 is a view showing the arrangement of the piezoelectric oscillators.

Figure 5 is a graph showing a plot of the sound pressure versus frequency characteristic of a piezoelectric oscillator.

Figure 6 is a graph showing a plot of the sound pressure versus frequency characteristic of a piezoelectric oscillator.

Figure 7 is a graph illustrating fluctuation of the length of time during which ultrasonic waves are generated and the length of the intervals between times when ultrasonic waves are generated.

Figure 8 is a diagrammatic view showing the external appearance of an remote controller.

Figure 9 is a frontal view showing the external appearance of the indoor unit of an air conditioner in accordance with a second embodiment.

Figure 10 is a right side view showing the external appearance of the indoor unit of an air conditioner in accordance with the second embodiment.

### Preferred Embodiments of the Invention

### [First Embodiment]

### <Overview of the Air Conditioner>

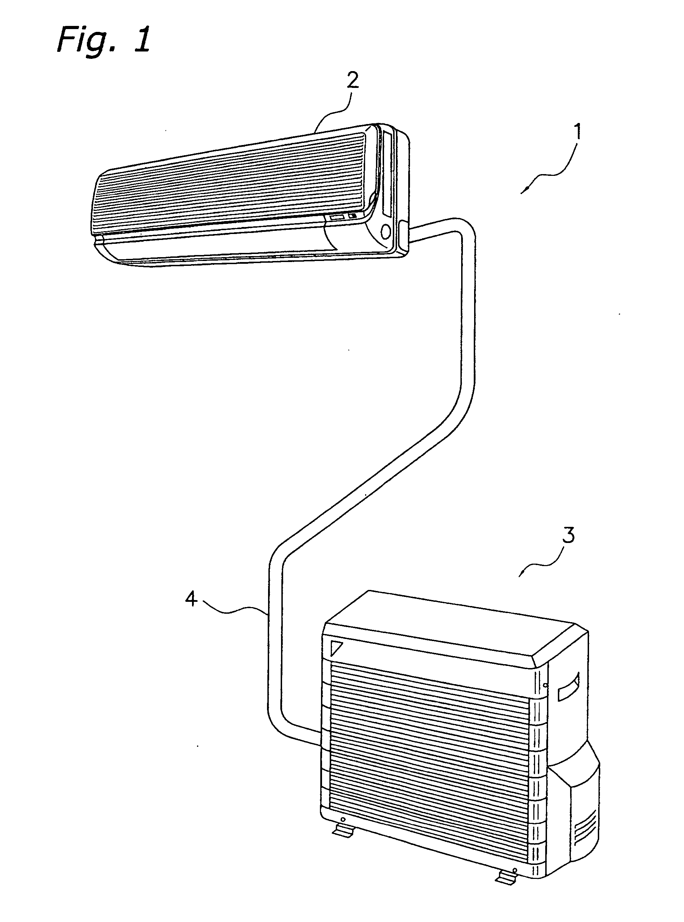

Figure 1 shows an air conditioner 1 that employs an embodiment of the present invention.

The air conditioner 1 is a device configured to condition the air inside a room by blowing into the room air that has been conditioned by being heated or cooled and humidified or dehumidified.

The air conditioner 1 includes an indoor unit 2 that is mounted to a wall or the like inside the room and an outdoor unit 3 that is installed outdoors. The outdoor unit 3 comprises an outdoor heat exchanger, an outdoor fan, an electronic component box, and other components (not shown) all enclosed in a casing.

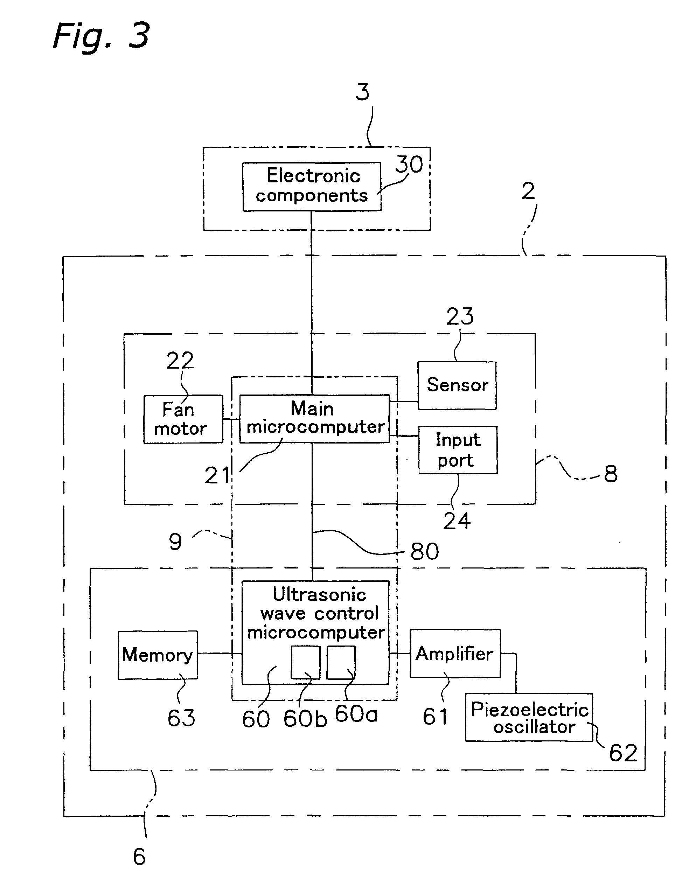

The indoor unit 2 includes an indoor heat exchanger, an indoor fan, a fan motor for driving the indoor fan, etc. (not shown), and the indoor heat exchanger and outdoor heat exchanger are connected by refrigerant piping 4 so as to form a refrigerant circuit. The indoor unit 2 is equipped with a control unit 9 and an ultrasonic wave generator 6 (see Figure 3). The control unit 9 controls such air conditioning functions as heating, cooling, and humidifying/dehumidifying and also controls the ultrasonic wave generating function. It controls the ultrasonic wave generator 6 in such a manner as to cause the ultrasonic waves generated by the ultrasonic wave generator 6 to overlap with the air blowing sound of the air conditioner 1. The control unit 9 comprises chiefly a main microcomputer 21 configured to control such air conditioning functions as heating, cooling, and humidifying/dehumidifying and an ultrasonic wave control microcomputer 60 configured to control the ultrasonic wave generator 6 (See Figure 3).

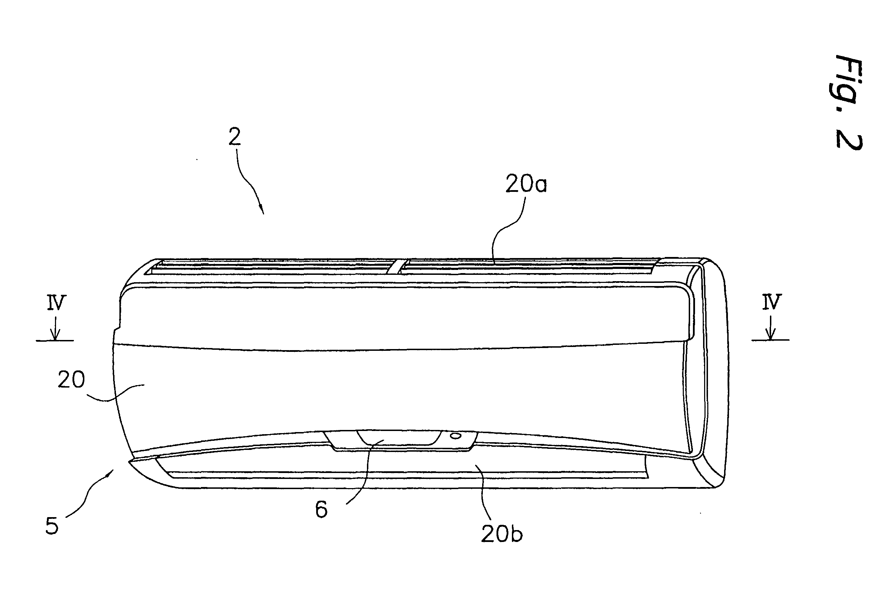

Figure 2 shows the external appearance of the indoor unit 2. The casing 5 of the indoor unit 2 comprises chiefly a front panel 20 that covers the top, bottom and sides of the indoor unit 2 from the front and a bottom frame (not shown) that is positioned toward the rear. A multitude of slit-shaped openings serving as intake vents 20a are provided in the top side of the front panel 20. An outlet vent 20b for discharging conditioned air is provided in a bottom front portion of the front panel 20. The ultrasonic wave generator 6 is provided above the outlet vent 20b in close proximity to the outlet vent 20b.

### <Constituent Features of the Ultrasonic Wave Generator>

The ultrasonic wave generator 6 is configured to generate ultrasonic waves having frequencies ranging from 20 kHz to 100 kHz. The constituent features of the ultrasonic wave generator 6 are shown in Figure 3. The ultrasonic wave generator 6 comprises chiefly the ultrasonic wave control microcomputer 60, an amplifier 61, a piezoelectric oscillator 62, and a memory 63 (discussed later).

The ultrasonic wave control microcomputer 60 has an ultrasonic signal generating circuit 60a configured to generate signals at ultrasonic frequencies of 20 kHz and above. The ultrasonic wave control microcomputer 60 is connected to the main microcomputer 21 and turns the ultrasonic wave generating function on and off based on control instructions received from the main microcomputer 21. The main microcomputer 21 is connected to a fan motor 22, various sensors 23 (e.g., a room temperature sensor and a humidity sensor), an electronic component 30 of the outdoor unit 3, and an input port 24, etc. for receiving signals from a remote controller (discussed later). The main microcomputer 21 controls the heating, cooling, humidifying/dehumidifying, and other air conditioning functions of the air conditioner 1.

The amplifier 61 is connected to the ultrasonic wave control microcomputer 60 and serves to amplify and output the voltage of the ultrasonic frequency signal it receives from the ultrasonic wave control microcomputer 60.

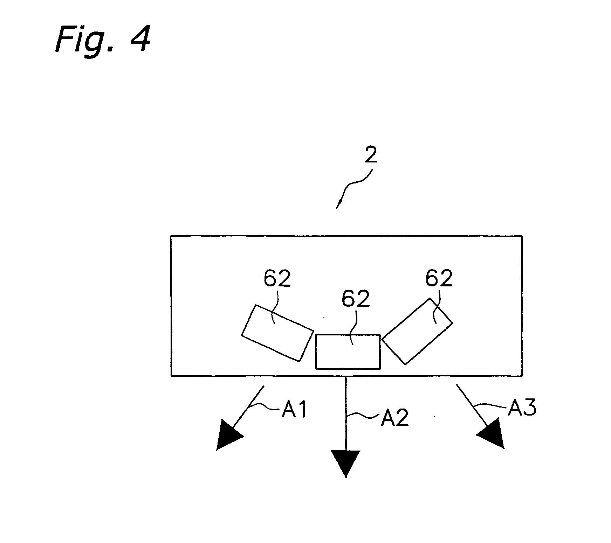

The piezoelectric oscillator 62 is configured to convert the amplified signal outputted from the amplifier 61 into ultrasonic waves. The piezoelectric oscillator 62 has a sound emitting body that vibrates and generates sound waves when an AC voltage is applied thereto. When a voltage having an ultrasonic frequency is applied to the piezoelectric oscillator 62, the sound emitting body vibrates at the ultrasonic frequency and generates ultrasonic waves. The piezoelectric oscillator 62 is arranged above the outlet vent 20b of the indoor unit in such a manner that it faces into the room and sends the generated ultrasonic waves toward the inside the room. As shown in Figure 4, this ultrasonic wave generator 6 has a plurality of piezoelectric oscillators 62 arranged facing in different directions such that they are mounted at slightly different angles in a horizontal plane. Figure 4 is a simplified cross sectional view of the arrangement of the piezoelectric oscillators 62 taken along line IV-IV of indoor unit 2 shown in Figure 2. Ultrasonic waves have strong directionality and are thus difficult to spread over a wide area. However, by arranging the piezoelectric oscillators 62 to emit ultrasonic waves in the multiple directions indicated by arrows A1, A2, and A3, ultrasonic waves can be delivered to a wide area. It is also acceptable to angle the piezoelectric oscillators 62 in the vertical direction.

The ultrasonic wave generator 6 is configured as a separate unit from the other devices of the air conditioner 1. More specifically, the ultrasonic wave generator 6 is physically separate from the air conditioner main unit 8, which includes the main microcomputer, and the ultrasonic wave control microcomputer 60 and the main microcomputer 21 are connected by a connector 80. The ultrasonic wave generating function of the ultrasonic wave generator 6 is turned on and off by the main microcomputer 21.

Since the ultrasonic wave generator 6 is a separate unit, it can be retrofitted into an existing air conditioner that does not have an ultrasonic wave generating function, enabling an ultrasonic wave generating function to be added easily and inexpensively.

The ultrasonic wave control microcomputer 60 also has an audible signal generating circuit 60b configured to generate a signal at an audible frequency. The ultrasonic wave control microcomputer 60 can switch between generation of a signal in the ultrasonic region and generation of a signal in the audible region based on a switching signal from the main microcomputer 21.

Thus, with this air conditioner 1, the piezoelectric oscillator 62 can be inspected during production of the indoor unit 2 and/or the ultrasonic wave generator 6 by sending a signal having an audible frequency to the piezoelectric oscillator 62. As a result, advanced ultrasonic inspection equipment is not required to inspect the piezoelectric oscillator 62 and the inspection can be accomplished more easily. Furthermore, with this air conditioner 1, the equipment used for mass production of the indoor unit 2 and/or the ultrasonic wave generator 6 can be simplified, incorrect inspection results can be reduced, and the time required to complete the inspection can be shortened.

### <Fluctuation Control>

It is said that the pulse of the human heart and other biological rhythms are generally undergoing 1/f fluctuations. While there are various fluctuations that take place in the natural world, it has been demonstrated that those fluctuations sharing the same 1/f quality as biological rhythms -- e.g., wind movements, the annual rings of trees, the flame of a candle, and the sound of waves -- impart a pleasant feeling to humans in terms of the senses of sight, hearing, and touch. In this air conditioner 1, l/f fluctuations are imparted to the flow rate or flow speed of the air blown out of the air conditioner 1 and l/f fluctuations are also imparted to a characteristic of the ultrasonic waves generated by the ultrasonic wave generator 6. The fluctuation of the characteristic of the ultrasonic waves is synchronized with the fluctuation of the air flow.

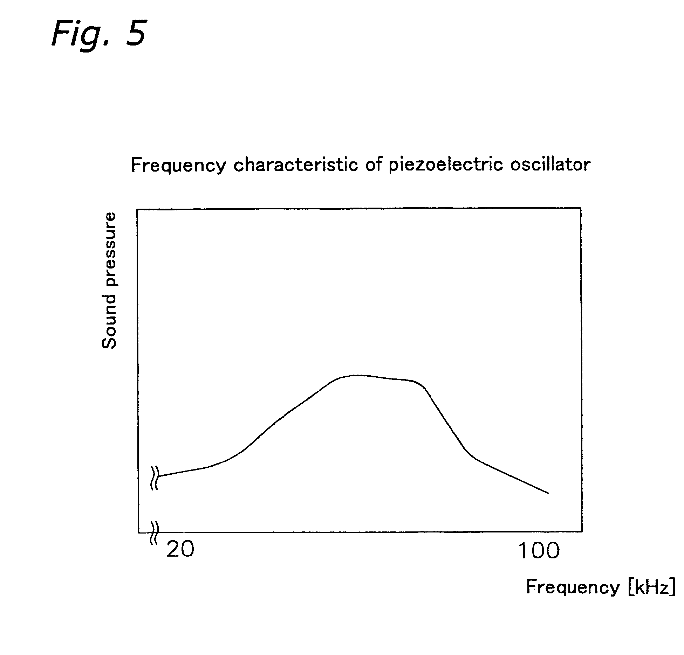

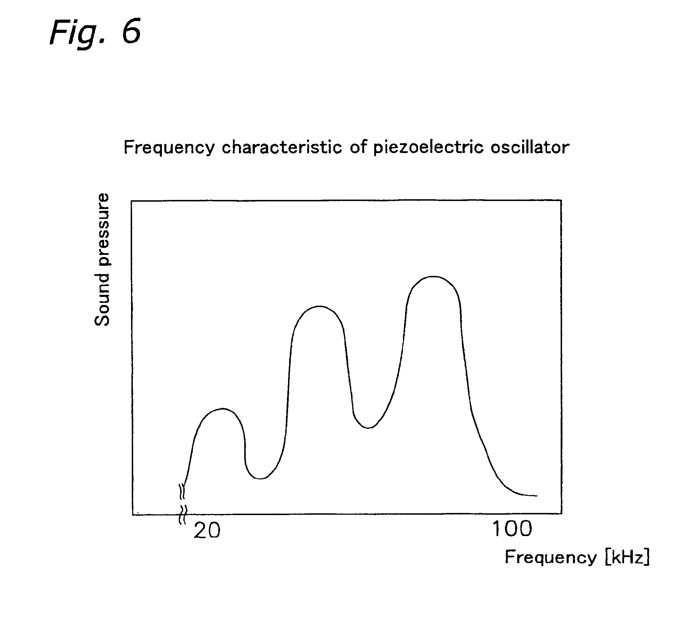

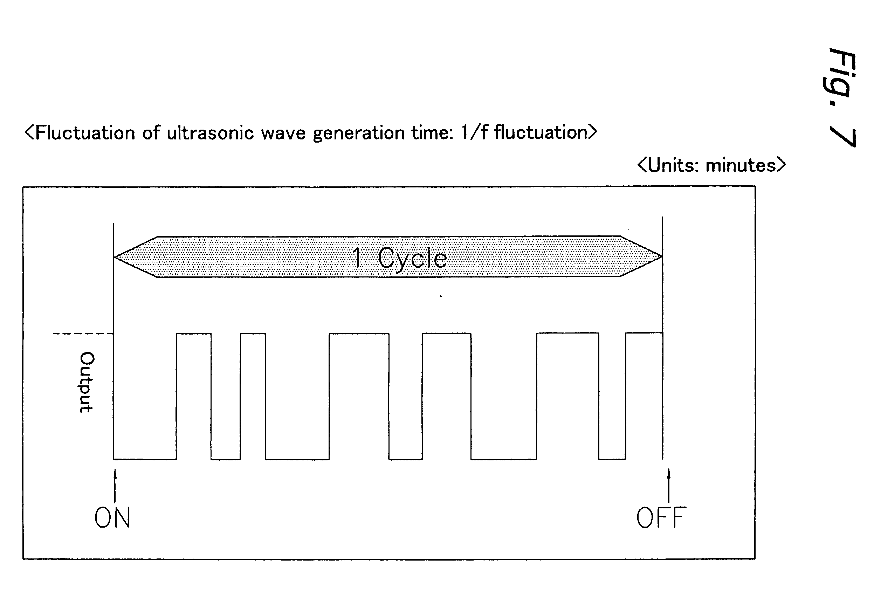

The ultrasonic wave characteristics to which 1/f fluctuation is imparted include time, frequency, and sound pressure and the following means exist for controlling the fluctuation of these characteristics.
[1] The frequency of the ultrasonic waves can be varied in a continuous manner between 20 kHz and 100 kHz using an ultrasonic wave control microcomputer 60 configured to provide any desired oscillation frequency.
   This method makes it possible to use fewer component elements than a case in which a plurality of elements each having a different natural frequency are used in order to generate ultrasonic waves at a plurality of frequencies and the number of elements is the same as the number of frequencies at which ultrasonic waves are to be generated. Thus, this ultrasonic wave generator 6 makes it possible to generate ultrasonic waves at a plurality of frequencies with an inexpensive device.
[2] The fact that the frequency characteristic of the piezoelectric oscillator 62 is not uniform can be utilized. As shown in Figure 5, the sound pressure of the sound waves generated by the piezoelectric oscillator 62 is not constant but, instead, various in a continuous manner with the variation of the frequency. Thus, the sound pressure can be made to fluctuate by fluctuating the frequency. As shown in Figure 6, it is also feasible to use a piezoelectric oscillator 62 having such a frequency characteristic that a plurality of sound pressure peaks exist within the frequency band of the ultrasonic waves generated. In such a case, if the frequency of the ultrasonic waves varies through one round trip across the frequency band, the sound pressure of the ultrasonic waves will also change repeatedly by a number of times equal to the number of peaks. In other words, the sound pressure can be varied several times by merely varying the frequency by a small amount. Thus, the sound pressure can be easily made to fluctuate. This kind of frequency characteristic is exhibited by some piezoelectric oscillators 62 that are normally intended for use in the audible region and, thus, such piezoelectric oscillators can be used in the ultrasonic wave generator 6. By using an audible region piezoelectric oscillator 62 of the sort often used in consumer electric appliances, the ultrasonic wave generator 6 can be fabricated inexpensively.
[3] By using an amplifier 61 whose amplification factor can be controlled by the ultrasonic wave control microcomputer 60, the sound pressure of the generated ultrasonic waves can be made to fluctuate by varying the amplification factor.
[4] As shown in Figure 7, the length of time during which ultrasonic waves are generated and the length of the intervals between times when ultrasonic waves are generated can be varied and fluctuated.
   The control means [1] to [4] described heretofore can be implemented by using software to control the ultrasonic wave control microcomputer 60 so as to impart l/f fluctuations to the sound pressure, frequency, and generation time of the generated ultrasonic waves.
   It is also acceptable to adopt the following control method.
[5] By using an ultrasonic wave control microcomputer 60 that can vary the frequency in any desired manner, ultrasonic waves of various frequencies can be generated in a short period of time by varying the frequency in a dispersed manner at a high speed based on pseudo random numbers generated by the ultrasonic wave control microcomputer 60. In this embodiment, the timing at which the frequency is varied is equal to or longer than 1 millisecond and shorter than or equal to 1 second.

With this control method, ultrasonic wave components in various frequency bands can be emitted without using a plurality of ultrasonic wave generators 6 and a state that approximates the natural world (in which sounds of various different frequencies are mixed together) can be created inexpensively. In short, an aural environment that approximates a natural environment can be recreated indoors inexpensively and it is possible to make the room more pleasant for the occupants.

It is difficult to compose a pulse if the timing at which the frequency is varied is shorter than 1 millisecond. If the timing is longer than 1 second, the timing at which the frequency of the ultrasonic waves is varied will become slow and it will be difficult to approximate a natural state.

### <Remote Controller>

With this air conditioner 1, such air conditioning functions as heating, cooling, and humidifying/dehumidifying and the ultrasonic wave generating function of the ultrasonic wave generator 6 can be controlled using a remote controller 7 configured to send control signals to the air conditioner 1.

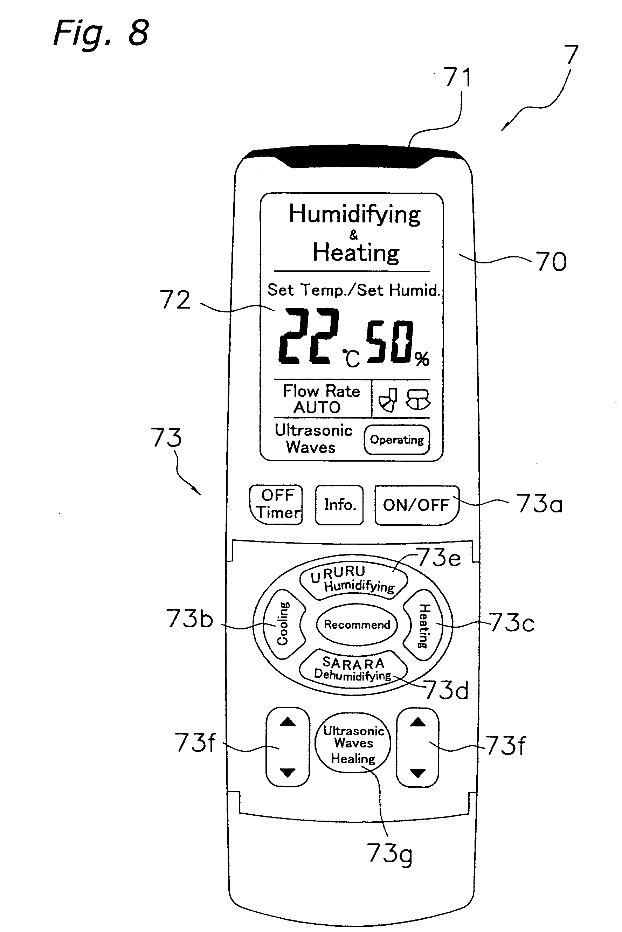

As shown in Figure 8, the remote controller 7 is provided with a remote controller main body 70, an output port 71, a liquid crystal display 72, and operating buttons 73.

The output port 71 is provided on the upper end of the remote controller main body 70 and is configured to send control signals to an input port 24 provided on the air conditioner 1.

The liquid crystal display 72 is provided on the front face of the remote controller 7. The liquid crystal display 72 is configured to display a screen indicating the content of button operations and a screen indicating such setting information as the temperature setting, the humidity setting, the fan speed setting, and the ultrasonic wave function setting.

The operating buttons 73 are provided on the front face of the remote controller 7 below the liquid crystal display 72. An occupant can select the details of how the air conditioner main unit 8 and the ultrasonic wave generator 6 will be controlled by pressing the operating buttons 73. The operating buttons 73 comprise an ON/OFF button 73a, a cooling mode button 73b, a heating mode button 73c, a dehumidifying mode button 73d, a humidifying mode button 73e, arrow buttons 73f, and an ultrasonic wave treatment button 73g. An occupant can accomplish various operations by pressing these buttons. In particular, the ultrasonic wave generation function can be turned on and off by simply pressing the ultrasonic wave treatment button 73g. An occupant can select the desired fluctuation pattern from among a number of pre-registered ultrasonic wave fluctuation patterns by using the arrow buttons 73f (fluctuation pattern selecting means) in combination with each other. The remote controller 7 transmits pattern identification data corresponding to the selected pattern from the output port 71 to the indoor unit 2 of the air conditioner 1. Inside the ultrasonic wave generator 6 of the indoor unit 2 is provided a memory 63 that stores data for a plurality of ultrasonic wave fluctuation patterns and pattern identification data assigned to each pattern (See Figure 3). When the indoor unit 2 receives the selected pattern identification data from the remote controller 7, the ultrasonic wave control microcomputer 60 reads the pattern data corresponding to the pattern identification data received from the remote controller 7 and controls the ultrasonic wave generator 6 accordingly.

With this remote controller 7, instead of transmitting a signal containing data expressing the entire selected ultrasonic wave fluctuation pattern, the remote controller 7 transmits pattern identification data corresponding to the selected ultrasonic wave fluctuation pattern. As a result, the volume of data in the transmitted signal is smaller than a case in which the transmitted signal contains data expressing the entire ultrasonic wave fluctuation pattern.

It is also acceptable to provide the remote controller 7 with a touch screen liquid crystal display wherewith various operations can be executed by touching the liquid crystal display at positions where the images of buttons are displayed instead of pressing operating buttons 73.

### <Characteristic Features>

[1] With this air conditioner 1, an ultrasonic wave generator 6 is used to generate ultrasonic waves having frequencies ranging from 20 kHz to 100 kHz in such a manner that the ultrasonic waves overlap with the blowing sound of the air conditioner 1. As a result, the sound that reaches the ears of a room occupant includes both the audible blowing sound of the air conditioner and an ample quantity of high-frequency ultrasonic waves having frequencies of 20 kHz and higher. Thus, a room occupant exposed to this sound will experience increased alpha wave activity due to the hypersonic effect. Alpha waves are the brain waves that exhibit increased activity when a person relaxes. This air conditioner 1 has a relaxing effect on room occupants and causes occupants to experience an increased feeling of comfort.
   Also, since the attenuation of ultrasonic waves becomes more severe as the frequency increases, ultrasonic waves at frequencies exceeding 100 kHz do not easily reach room occupants. However, since the air conditioner 1 is configured such that the ultrasonic waves are generated at frequencies ranging from 20 kHz to 100 kHz, the ultrasonic waves can reach the room occupants and can thus impart the hypersonic effect to the room occupants.
[2] In this air conditioner 1, l/f fluctuations are imparted to the flow of air blown out of the air conditioner 1 and 1/f fluctuations are also imparted to a characteristic of the ultrasonic waves generated by the ultrasonic wave generator 6. When the cyclical frequencies of the fluctuations of the blowing sound and the ultrasonic waves are different, these sounds are heard as noise and could possibly result in an unpleasant feeling on the part of the room occupants. However, with this air conditioner 1, the characteristics of the ultrasonic waves are made to fluctuate at approximately the same cyclical frequency as the fluctuation of the blowing sound and, thus, such an unpleasant feeling can be prevented.
[3] With this air conditioner 1, such air conditioning functions as heating, cooling, and humidification/dehumidification are controlled by a main microcomputer 21 and the fluctuation of the ultrasonic waves is controlled by an ultrasonic wave control microcomputer 60. Consequently, the load associated with controlling the ultrasonic wave fluctuation can be prevented from being imposed on the main microcomputer 21 used to control the air conditioning functions.
[4] If the transmitter used to transmit ultrasonic waves toward the inside of a room is located deep inside the air conditioner 1, the components of the air conditioner 1 will act as obstacles and cause the ultrasonic waves to attenuate easily. As a result, it will be difficult for the ultrasonic waves to reach the room occupants.
   However, with this air conditioner 1, the piezoelectric oscillator 62 serving as the transmitter is arranged above the outlet vent 20b of the air conditioner 1 in such a manner as to face into the room. In other words, the piezoelectric oscillator 62 is arranged in a position where it has access to the space inside the room and the ultrasonic waves emitted from the piezoelectric oscillator 62 are not likely to be hindered by an obstacle before reaching the room occupants. In short, with this air conditioner 1, the ultrasonic waves easily reach the room occupants.
   Additionally, in order to obtain the hypersonic effect, it is necessary for the ultrasonic waves to be heard while being overlapped with audible sound. With this air conditioner 1, since the ultrasonic waves are generated in the vicinity of the outlet vent 20b where the blowing sound of the air conditioner 1 occurs, the ultrasonic waves readily overlap with the blowing sound and the hypersonic effect can be obtained with ease. It is also acceptable to mount the piezoelectric oscillator 62 on the outside surface of the front panel 20 near the intake vent 20a, for example. This kind of position, too, would give the piezoelectric oscillator 62 access to the room space with few obstacles to hinder the ultrasonic waves, allowing the same effects as described above to be obtained.
[5] With this air conditioner 1, a conventional microcomputer configured for use with a wireless remote controller can be used as the ultrasonic wave control microcomputer 60 and an audible region piezoelectric oscillator of the sort used in consumer electric appliances can be used as the piezoelectric oscillator 62. As a result, an ultrasonic wave function can be obtained with a combination of existing components and the manufacturing cost can be held in check.

### [Second Embodiment]

### <Positioning of the Ultrasonic Wave Generator>

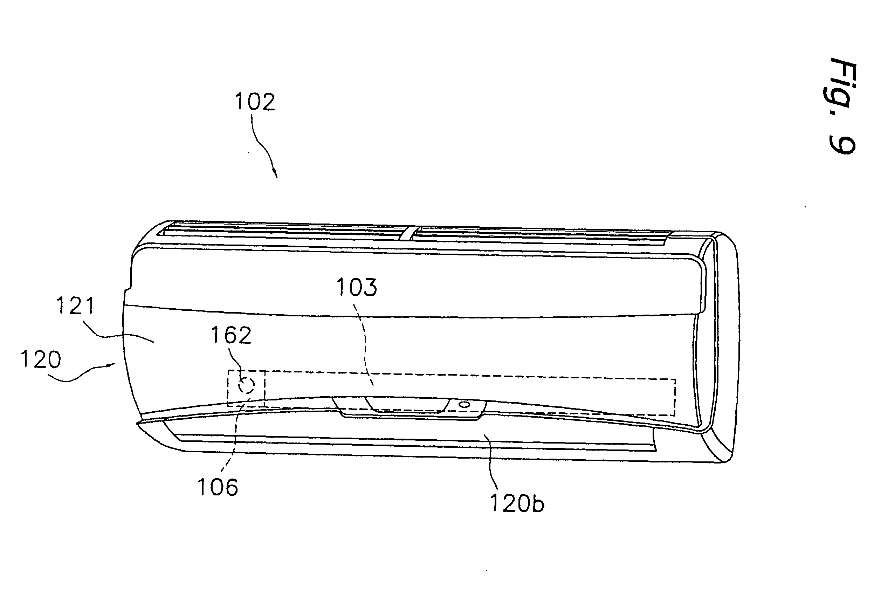

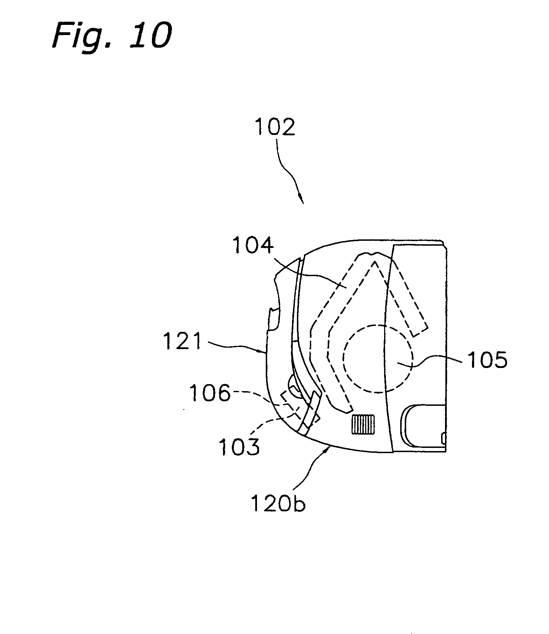

Figures 9 and 10 show the external appearance of the indoor unit 102 of an air conditioner that employs a second embodiment of the present invention. Figure 9 is a frontal view of the indoor unit 102 and Figure 10 is a right-hand side view of the indoor unit 102.

This indoor unit 102 is characterized by the arrangement of the ultrasonic wave generator 106 and the arrangement of the ultrasonic wave generator 106 will now be described. Although the other constituent features are the same as the indoor unit 2 of the air conditioner 1 of the first embodiment, the different reference numerals are used in the figures illustrating the second embodiment than are used for the same features in the figures illustrating the first embodiment.

As shown in Figure 9, the front panel 120 of the indoor unit 102 has a front section 121 that covers the front of the indoor unit 102. With this indoor unit 102, an electronic component unit 103 is provided above but near the outlet vent 120b inside a lower portion of the front section 121. The electronic component unit 103 comprises printed circuit boards containing control circuits and an electronic component box housing the printed circuited boards and is elongated in the lengthwise direction of the indoor unit 102.

With this indoor unit 102, the ultrasonic wave generator 106 is positioned in closely adjacent to the electronic component unit 103 and on the left side of the electronic component unit 103 in a frontal view. The ultrasonic wave generator 106 has a piezoelectric oscillator 162.

As shown in Figure 10, the electronic component unit 103 and the ultrasonic wave generator 106 are arranged facing the back side of a lower portion of the front section 121 and between the lower portion of the front section 121 and the indoor heat exchanger 104. The ultrasonic wave generator 106 is also disposed in such an orientation that it emits the ultrasonic waves forward and slightly downward from the indoor unit 102. The indoor heat exchanger 104 is mounted in such a manner as to surround the frontal, upper, and upper rear portions of an indoor fan 105 that is arranged near the center of the indoor unit 102 in a side view.

Instead of arranging the entire ultrasonic wave generator 106 on the back side of a lower portion of the front section 121, it is also acceptable to arrange only the piezoelectric oscillator 162 in such a position.

### <Characteristic Features>

With this air conditioner, an ultrasonic wave generator 106 having a piezoelectric oscillator 162 configured to transmit ultrasonic waves into a room is arranged facing the back side of the front section 121. Thus, the piezoelectric oscillator 162 is arranged in one of the more frontward positions inside the air conditioner. Air conditioners are generally arranged such that the living space of the room is positioned in front of the air conditioner. Thus, with this air conditioner, the piezoelectric oscillator 162 is arranged in a position that is closer to the room occupants than any other component inside the indoor unit 102. Since the piezoelectric oscillator 162 is arranged farther forward than the heat exchanger 104, there are few objects (such as the heat exchanger 104) in front of the piezoelectric oscillator 162 that can obstruct the ultrasonic waves. In short, with this air conditioner, the ultrasonic waves can be made to reach the room occupants more plentifully.

### [Other Embodiments]

[1] It is also acceptable for the ultrasonic wave generator 6 to use a combination of wave transmitters having different frequency characteristics. When ultrasonic waves from a wide band of frequencies are to be generated and the desired range of frequencies cannot be generated with a single ultrasonic wave transmitter, a wide band of frequencies can be covered by using a combination of wave transmitters having different frequency characteristics.
   In such a case, it is good to use a combination of general-purpose wave transmitters that are very commonplace. For example, if it is desired to generate ultrasonic waves in a frequency band ranging from 20 kHz to 60kHz, then a good approach would be to use a piezoelectric element intended for use as a buzzer at frequencies in the vicinity of 20 kHz and a piezoelectric element intended to be used for distance measuring at frequencies in the vicinity of 40 kHz. With this approach, the elements are widely available and inexpensive. Consequently, the cost of the ultrasonic wave generator 6 can be reduced.
[2] Although ultrasonic waves ranging from 20 kHz to 100 kHz are generated in the embodiments described above, it is also acceptable to generate ultrasonic waves at other frequencies as necessary depending on the situation. For example, if the room is large and it is necessary to transmit the ultrasonic waves over a long distance or if it is necessary to use a narrower band of frequencies in order to curb the cost of the ultrasonic wave generator, the upper limit of the frequency can be reduced by using a frequency range of 20 kHz to 50 kHz or the like.
[3] Although the air conditioner 1 described heretofore is an example of a device configured to condition the air of a room by delivering air that has been heated/cooled and humidified/dehumidified into the room, it is also acceptable to provide an ultrasonic wave generator 6 in an air cleaning device configured to deliver air that has had dust, particles, and odors removed therefrom into a room. In the case of such an air cleaning device, too, the comfort of the room occupants can be improved by generating ultrasonic waves. It is also acceptable for the air conditioner to be a floor unit instead of a wall-mounted unit. In the case of a floor unit, too, the comfort of the room occupants can be improved by delivering ultrasonic waves to the room occupants.

### Applicability to Industry

By utilizing the present invention, the comfort of room occupants can be improved by delivering to the occupants ultrasonic waves that are capable of imparting a hypersonic effect.

## Claims

1. An air conditioner (1) that is configured to blow conditioned air into a room, the air conditioner being equipped with:
an ultrasonic wave generator (6, 106) configured to generate ultrasonic waves having frequencies ranging from 20 kHz to 100 kHz; and
a control unit (9) configured to control the ultrasonic wave generator (6, 106) such that the ultrasonic waves overlap with the air blowing sound of the air conditioner.

2. The air conditioner described in claim 1, wherein the control unit (9) executes control in such a manner that a characteristic of the ultrasonic waves fluctuates.

3. The air conditioner described in claim 2, wherein the control unit (9) executes control in such a manner that
the flow rate or flow speed of the air blown into the room fluctuates and
a characteristic of the ultrasonic waves fluctuates at approximately the same cyclical frequency as the fluctuation of the blowing sound of the air conditioner (1).

4. An air conditioner as described in claim 2 or 3, wherein the characteristic is the frequency of the ultrasonic waves.

5. An air conditioner as described in claim 4, wherein
the control unit (9) executes control in such a manner that the frequency of the ultrasonic waves changes at intervals of I millisecond, 1 second, or an amount of time between 1 millisecond and 1 second.

6. An air conditioner as described in claim 2 or 3, wherein the characteristic is the sound pressure of the ultrasonic waves.

7. An air conditioner as described in claim 6, wherein
the ultrasonic wave generator (6, 106) is equipped with
an oscillator (60a) configured to generate a signal having an ultrasonic frequency,
an amplifier (61) configured to amplify the voltage of the signal, and
a piezoelectric element (62, 162) configured to convert the amplified signal
outputted from the amplifier (61) into ultrasonic waves; and
the controller (9) varies the amplification factor of the amplifier (61).

8. An air conditioner as described in claim 4 or 5, wherein
the ultrasonic wave generator (6, 106) is equipped with a piezoelectric element (62, 162) having a plurality of sound pressure peaks within the frequency band where the frequency of the ultrasonic waves is made to fluctuate by the control unit (9).

9. An air conditioner as described in any one of claims 2 to 8, wherein the controller (9) has:
a first control unit (21) configured to control the air conditioning functions of the air conditioner (1); and
a second control unit (60) configured to control the fluctuation of the ultrasonic waves of the ultrasonic wave generator (6, 106).

10. An air conditioner as described in any one of claims 1 to 9, wherein
the ultrasonic wave generator (6) has a wave transmitter (62) configured to transmit the ultrasonic waves toward the inside of the room; and
the wave transmitter (62) is arranged in a position where it has access to the space inside the room.

11. An air conditioner as described in any one of claims 1 to 9, wherein
the air conditioner is further provided with a casing (120) having a front face part (121) that covers the front face of the air conditioner;
the ultrasonic wave generator (106) has a wave transmitter (162) configured to transmit ultrasonic waves toward the inside of the room; and
the wave transmitter (162) is arranged near the rear side of the front face part (121).

12. An air conditioner as described in claim 1 or 9, wherein
the air conditioner is further provided with
a casing (120) having a front face part (121) that covers the front face of the air conditioner and
an indoor heat exchanger configured to exchange heat with the air inside the room;
the ultrasonic wave generator (106) has a wave transmitter (162) configured to transmit the ultrasonic waves toward the inside of the room; and
the wave transmitter (162) is arranged between the front face part (121) and the heat exchanger (104).

13. An air conditioner as described in any one of claims 10 to 12, wherein
the air conditioner (1) is further provided with an outlet vent (20b, 120b) through which conditioned air is blown out; and
the wave transmitter (62, 162) is arranged near the outlet vent (20b, 120b).

14. A remote controller (7) configured to send control signals to an air conditioner (1) as described in any one of claims 1 to 13, the remote controller being provided with a control selecting means (73g) configured to allow a user to select how the ultrasonic wave generator (6, 106) will be controlled.

15. A remote controller configured to send control signals to an air conditioner (1) that is configured as described in any one of claims 2 to 13 and also provided with a memory unit (63) for storing a plurality of patterns for fluctuation of the ultrasonic waves and pattern identification data corresponding to each of the patterns, the remote controller (7) being provided with
a fluctuation pattern selecting means (73) configured to allow a user to select the pattern; and
a signal transmitter (71) configured to transmit pattern recognition data corresponding to the pattern selected using the fluctuation pattern selecting means (73).

16. An ultrasonic wave generating unit configured to be mounted to an air conditioner (1) that is configured to blow conditioned air into a room, the ultrasonic wave generating unit being equipped with:
an ultrasonic wave generator (6, 106) configured to generate ultrasonic waves having frequencies ranging from 20 kHz to 100 kHz; and
a control unit (60) configured to control the ultrasonic wave generator such that the ultrasonic waves overlap with the air blowing sound of the air conditioner.

17. An air conditioner as described in claims 7 or 8, wherein
the ultrasonic wave generator is further provided with an audible signal oscillator (60b) configured to generate a signal having an audible frequency.
